(19) Europäisches Patentamt
European Patent Office
Office européen des brevets

(11) Publication number: 0 299 382 A1

# (12) EUROPEAN PATENT APPLICATION

(21) Application number: 88110942.5

(22) Date of filing: 08.07.88

(51) Int. Cl.4: C07D 213/64 , A01N 43/40

Claims for the following Contracting State: ES.

(30) Priority: 14.07.87 JP 173888/87
05.04.88 JP 82143/88

(43) Date of publication of application:
18.01.89 Bulletin 89/03

(84) Designated Contracting States:
AT BE CH DE ES FR GB GR IT LI NL SE

(71) Applicant: MITSUBISHI PETROCHEMICAL CO., LTD.
5-2, 2-chome, Marunouchi
Chiyoda-ku Tokyo 100(JP)

(72) Inventor: Goh, Atsushi
1977-7, Kosaka-machi
Ushiku-shi Ibaraki-ken(JP)
Inventor: Imada, Satoshi
1-11-4-302, Chuo, Ami-machi
Inashiki-gun Ibaraki-ken(JP)
Inventor: Jikihara, Kazuo
1307-10, Onabake-machi
Ushiku-shi Ibaraki-ken(JP)
Inventor: Endo, Keiji
1-11-5-203, Chuo, Ami-machi
Inashiki-gun Ibaraki-ken(JP)
Inventor: Yamamoto, Yuri
1-11-5-204, Chuo, Ami-machi
Inashiki-gun Ibaraki-ken(JP)
Inventor: Usui, Yoshihiro
1-11-4-201, Chuo, Ami-machi
Inashiki-gun Ibaraki-ken(JP)

(74) Representative: Kraus, Walter, Dr. et al
Patentanwälte Kraus, Weisert & Partner
Thomas-Wimmer-Ring 15
D-8000 München 22(DE)

## (54) 2-nitro-5-(substituted pyridyloxy)benzohydroxymic acid derivatives.

(57) A compound of the formula

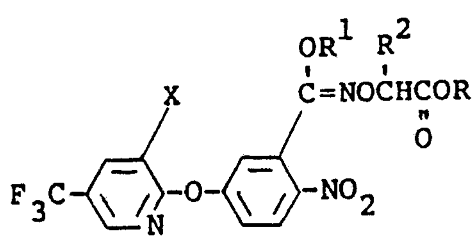

... (I)

in which R and R¹ each represent a lowre alkyl group, R²· represents a hydrogen atom or a lower alkyl

EP 0 299 382 A1

group, and X represents a hydrogen or a halogen atom; a process for its production; and its use as a herbicide.

This invention relates to novel 2-nitro-5-(substituted pyridyloxy)benzohydroximic acid derivatives.

More specifically, this invention pertains to a 2-nitro-5-(substituted pyridyloxy)benzohydroximic acid derivative represented by the following formula

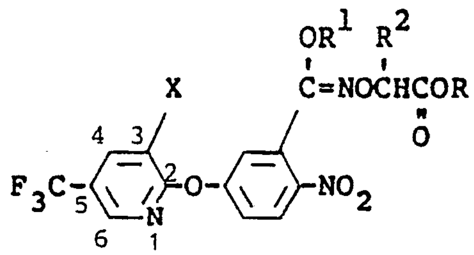

... (I)

in which R and $R^1$ each represent a lower alkyl group, $R^2$ represents a hydrogen atom or a lower alkyl group, and X represents a hydrogen or halogen atom;
a process for its production; and to a herbicide containing the above compound as an active ingredient.

The novel benzohydroximic acid derivative of formula (I) provided by this invention has very high herbicidal activity on weeds, for example in their pre-emergence and post-emergence stages, and the strongest herbicidal activity in their growth stage, and is useful as a herbicide.

European Patent Application EP-A-155613 discloses compounds of the general formula (A)

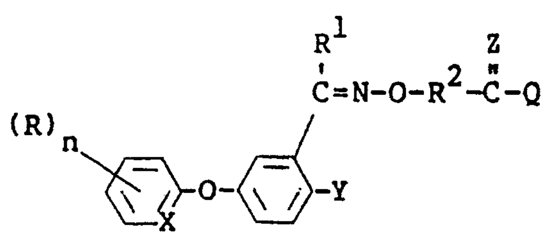

... (A)

as novel diphenyl ether oxime ester derivatives.

In general formula (A), X is CH or N; Y is a nitro group, halogen or a cyano group; Z is oxygen or sulfur; R is halogen or a nitro, cyano, alkyl, haloalkyl, alkylthio, haloalkylthio, alkoxy, haloalkoxy, sulfonamide, dialkylsulfonamide, alkylsulfonyl, haloalkylsulfonyl, alkylsulfinyl or haloalkylsulfinyl group; n is 1, 2 or 3; $R^1$ is a hydrogen or halogen atom, or a cyano, alkyl, haloalkyl, cyanoalkyl, alkoxy, haloalkoxy, cyanoalkoxy, alkylthio, haloalkylthio, cyanoalkylthio, mono-or di-alkylamino, alkylthioalkyl or mono- or di-alkylaminoalkyl group; $R^2$ is a $C_1$-$C_6$ alkylene or alkenyl group which may be substituted by an alkyl, haloalkyl, cyanoalkyl or hydroxyl group; Q is $-OR^3$ or $-SR^3$; and $R^3$ is an alkoxyalkyl group, a thioalkyl group, a cyanoalkyl group, a cycloalkyl group, a hydroxyalkyl group, a carboalkoxyalkyl group, an alkylthioalkyl group, an aralkyl group, a sulfonamide group, a 4- to 6-membered heterocyclic group having not more than 3 hetero atoms in the ring, or an alkyl group substituted by the heterocyclic group.

In the general formula (A) which could embrace a very large number of compounds, X may include N, R may include halogen and haloalkyl, Y may include $NO_2$, $R^1$ may include alkoxy, $R^2$ may include $C_1$ alkylene which may be substituted by alkyl, and Z may include an oxygen atom. However, the definition of $R^3$ in $-OR^3$ for Q in formula (A) does not include unsubstituted lower alkyl groups defined for R in the compounds of this invention represented by formula (I).

EP-A-155613 shows that compounds of formula (A) in which Q is $-OR^6$ ($R^6$ is a lower alkyl group) can be synthesis intermediates for the synthesis of the final compounds of formula (A) in which Q is $-OR^3$ by ester-interchange reaction. But the specification fails to describe specific examples of such intermediate compounds and does not describe nor suggest the herbicidal activity of such intermediates. Thus, EP-A-155613 does not at all disclose specific examples of the compounds of formula (I) provided by this invention which are not embraced by the compounds of formula (A), and naturally does not give any description or suggestion about their herbicidal activity as well as their biological activity.

Moreover, EP-A-155613 does not show any specific example of compounds of formula (A) in which $R^1$ is an alkoxy group or a substituted alkoxy group and their synthesis. $R^1$ in the compounds of formula (A) which were synthesized is only a methyl group. The above European Patent Application cites only alkyl and haloalkyl groups as preferred examples of $R^1$, and CH as a preferred example of X. Accordingly, EP-A-155613 totally fails to suggest a guideline for selecting the combination of these groups specified in the present invention. Evidently, it only recommends selection of different combinations of groups from those in the compounds of this invention represented by formula (I). In addition, EP-A-155613 has a general description to the effect that the compounds of formula (A) have herbicidal activity, but does not at all show any specific data by which their herbicidal activity can be determined.

Many herbicides have been developed and are used now for croplands or non-croplands. Among them, foliar treating-type non-selective herbicides are used in non-croplands such as railroads, factory sites, parks and vacant lands, or in croplands such as orchards, nurseries for forest trees and upland farms by a treatng method devised for avoiding contact with the leaves and stalks of useful plants by utilizing the property of the herbicides to be quickly inactivated in the soil by adsorption or decomposition and not to exert a deleterious effect on the growth of the useful plants via the soil.

In principal agricultural countries of the world, for example U. S. A., soil erosion of croplands by rainfalls, wind, etc. gives rise to a great problem, and in recent years as a measure of preserving the soil against erosion, the concervation tillage by which the number of plowings of the cropland is reduced or the non-tillage by which crops are sown without plowing has come into practice. In the case of non-tillage practice, weeds which grow before sowing have to be controlled, and non-selective herbicides of the foliar treating type are used for this purpose.

Now, 1,1′-dimethyl-4,4′-bipyridium dichloride (paraquat), N-(phosphonomethyl)glycine (Glyphosate), 2-amino-4-[(hydroxy) (methyl)phosphionyl]butyryl alanyl alanine sodium salt (Bialaphos), and ammonium-(3-amino-3-carboxypropyl)-methylphopshinate (Glufosinate ammonium) have been developed or used as the foliar treating-type non-selective herbicides. The number of these herbicides, however, is very small. Since there are many kinds of weeds that occur, and when weeds in various growth stages occur together, these existing agents are not insufficient in effect. It has been desired to develop chemicals which have a broader herbicidal spectrum and a higher efficacy.

The present inventors made extensive research efforts in order to overcome the technical problem of the foliar treating-type non-selective herbicides, and develop pyridyl phenyl ether type herbicides which permit low dosages, have a broad herbicidal spectrum, low residual activity in soil and low mammalian toxicity. These efforts have led to the discovery that the compounds of formula (I) given above have excellent herbicidal activity and can overcome the technical problem of the prior art, and that the compounds of formula (I) shows a broad herbicidal spectrum at reduced dosages.

Thus, according to this invention, there is provided a 2-nitro-5-(substituted pyridyloxy)benzo hydroximic acid derivative represented by the following formula (I)

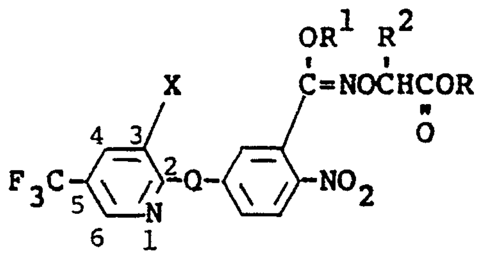

... (I)

in which R and $R^1$ each represent a lower alkyl group, $R^2$ represents a hydrogen atom or a lower alkyl group, and X represents a hydrogen or halogen atom.

In the present specification and the appended claims, the term "lower" used to qualify a group or a compound means that the group or compound so qualified has not more than 6, preferably not more than 4, carbon atoms.

The pyridyl phenyl ether derivative of general formula (I) provided by this invention is structurally characterized by the fact that a trifluoromethyl group is present at the 5-position of the pyridine ring, and hydroximic ester moiety defined in formula (I) is bonded to the benzene ring at the ortho-position to the nitro group. This structural characteristic is believed to be attributed to the excellent properties of a herbicide comprising the pyridyl phenyl ether derivative of formula (I).

The compound of this invention has a broad herbicidal spectrum in non-crop lands and croplands for rice, corn, wheat, barley, soybean, etc. and fully exhibits its herbicidal activity in much lower dosages than ordinary herbicides. These excellent properties cannot be expected from the prior art, and the compound of the invention is very useful as a herbicide.

In the pyridyl phenyl ether derivative of formula (I), the "halogen" atom may be, for example, fluorine,

chlorine, bromine or iodine. Fluorine and chlorine, especially the latter, are preferred.

The "lower alkyl group" may be a linear or branched alkyl group such as methyl, ethyl, n-propyl, iso-propyl, n-butyl, sec-butyl, iso-butyl, tert-butyl, n-amyl or iso-amyl.

In the compound of formula (I) provided by this invention, $R^1$ is preferably a methyl group, and $R^2$ is preferably a hydrogen atom or a methyl group, particularly the former. Conveniently, R is a methyl or ethyl group.

The substituent X at the 3-position of the pyridine ring is suitably a chlorine atom.

Especially preferred as the compound of formula (I) provided by this invention are methyl O-methoxycarbonylmethyl-5-(3-chloro-5-trifluoromethyl-2-pyridyloxy)nitrobenzohydroximate and methyl O-ethoxycarbonylmethyl-5-(3-chloro-5-trifluoromethyl-2-pyridyloxy)-2-nitrobenzohydroximate.

The compound of formula (I) has an asymmetric carbon atoms, and may exist as a dextrorotatory, levorotatory or racemic form. The compound of formula (I) includes two stereoisomers represented by formulae (I-a) and (I-b) [syn-type and anti-type]. It should be understood that formula (I) encompasses both of these stereoisomers

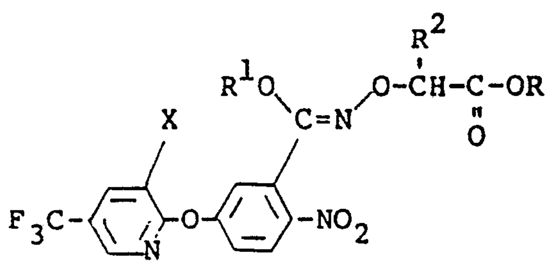

(I-a)

(syn-type)

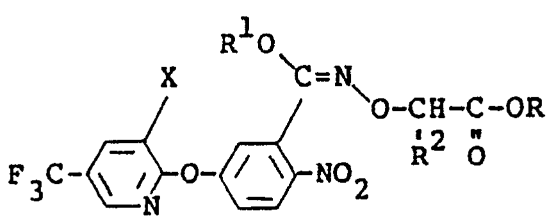

(I-b)

(anti-type)

There is no great difference in herbicidal activity between the two stereoisomers, but the syn-type stereoisomer is generally preferred from the standpoint of mutagenicity.

The compound of this invention can be produced by a process shown by the following reaction scheme A.

Reaction Scheme A

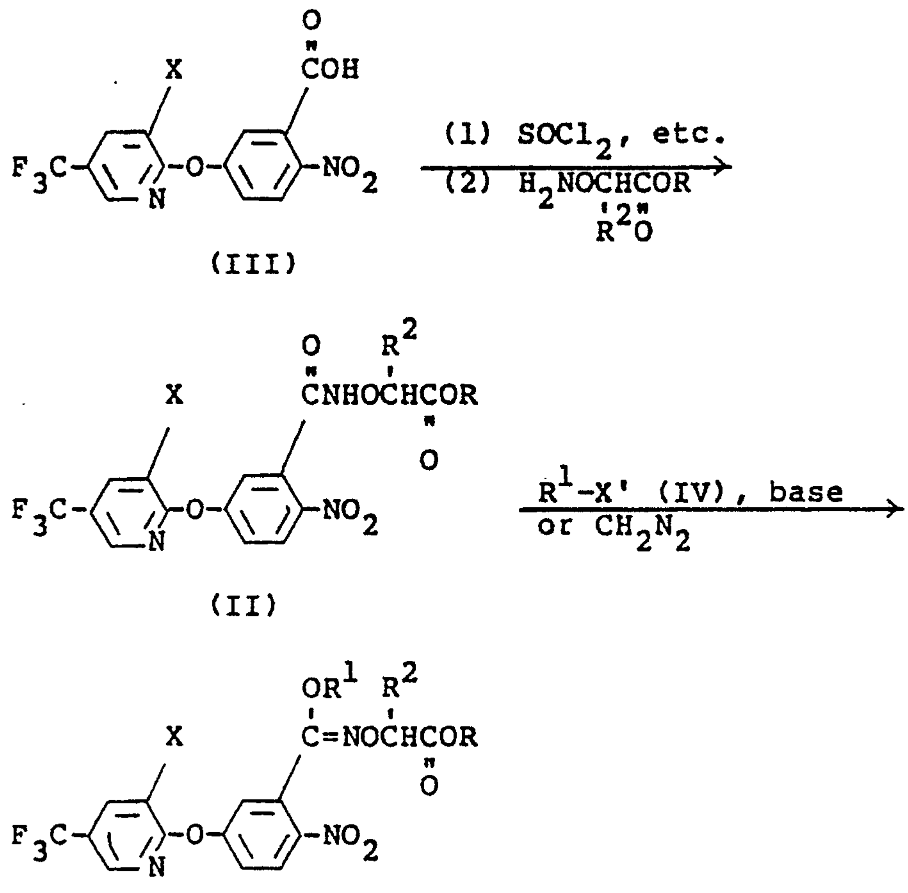


(III)

(II)

(I)

In the above reaction scheme, X, $R^1$, $R^2$ and R are as defined in formula (I). X′ represents a halogen atom or the group-$OSO_2R'$ in which R′ represents a substituted or unsubstituted alkyl, phenyl or alkoxy group.

According to this embodiment, the hydroxamic acid derivative (II) can be obtained by converting the 2-nitro-5-(substituted pyridyloxy)benzoic acid (III) to an acid chloride with, for example, thionyl chloride, and reacting the acid chloride with an O-substituted hydroxylamine, for example in an organic solvent in the presence of a base.

In the reaction, the amount of thionyl chloride used may be properly changed. For example, it may be used in an amount of 1 to 10 equivalents to the compound of formula (III). The reaction temperature may also be properly chosen, and is, for example, room temperature to 80 °C. In the reaction of the acid chloride with the O-substituted hydroxylamine, the amount of the O-substituted hydroxylamine used may be properly chosen, for example 1 to 3 equivalents, preferably 1 to 1.5 equivalents. The amount of the base can also be properly selected, and is, for example, 1 to 3 equivalents, preferably 1 to 1.5 equivalents. The reaction temperature at this time may be, for example, -10 to 100 °C, preferably 0 °C to room temperature.

Examples of the solvent used include aromatic hydrocarbons such as benzene and toluene, ethers such as tetrahydrofuran and dioxane, acetone, acetonitrile, dimethyl formamide and dimethyl sulfoxide. A mixture of such an organic solvent with water may also be used. Examples of the base are pyridine, triethylamine, sodium carbonate, potassium carbonate and sodium hydrogen carbonate.

The resulting O-substituted-2-nitro-5-(substituted pyridyloxy)benzohydroxamic acid derivative (II) is reacted with an alkylating agent (IV) or $CH_2N_2$ in an organic solvent in the presence or absence of a base to produce the hydroximic acid derivative (I) used as a herbicide in this invention.

The reaction may be carried out by using 1 to 3 molar equivalents, preferably 1 to 1.5 molar equivalents, of the compound (IV) or $CH_2N_2$ with respect to the compound (II) at a temperature of 0 °C (under cooling with ice) to the refluxing temperature of the solvent, preferably room temperature to about 80 °C, for a period of 0.5 to 20 hours. Examples of the solvent used in this reaction are alcohols such as methanol and ethanol, aromatic hydrocarbons such as benzene and toluene, ethers such as diethyl ether, tetrahydrofuran and dioxane, acetone, acetonitrile, dimethyl formamide, and dimethyl sulfoxide. A mixture of such an organic solvent and water may also be used.

Examples of the base used in the above reaction include pyridine, triethylamine, sodium hydroxide, potassium hydroxide, sodium carbonate, potassium carbonate, sodium ethylate, and sodium hydride. An acid binder may be used in an amount of, for example, 1 to 3 equivalents, preferably 1 to 1.5 equivalents, to the compound (IV). The use of the acid binder is preferred because it leads to good yields. When the reaction is carried out using $CH_2N_2$ as the alkylating agent, no base needs to be used.

When the reaction is carried out in a two-phase system, it is possible to use a phase transfer catalyst, for example a quaternary ammonium salt such as tetramethyl ammonium bromide, tetrabutyl ammonium bromide or benzyl tributyl ammonium bromide and a quaternary phosphonium such as tetraphenyl phosphonium bromide in an amount of, for example, 1 to 50 % by weight, preferably 5 to 30 % by weight.

After the reaction, the compound of the invention can be isolated by, for example, pouring the reaction mixture in water, and treating it in a customary manner, for example by extraction with an organic solvent, re-crystallization or column chromatography.

The compound of formula (I) of this invention may also be synthesized by a process shown by the following reaction scheme B.

Reaction Scheme B

[1st step]

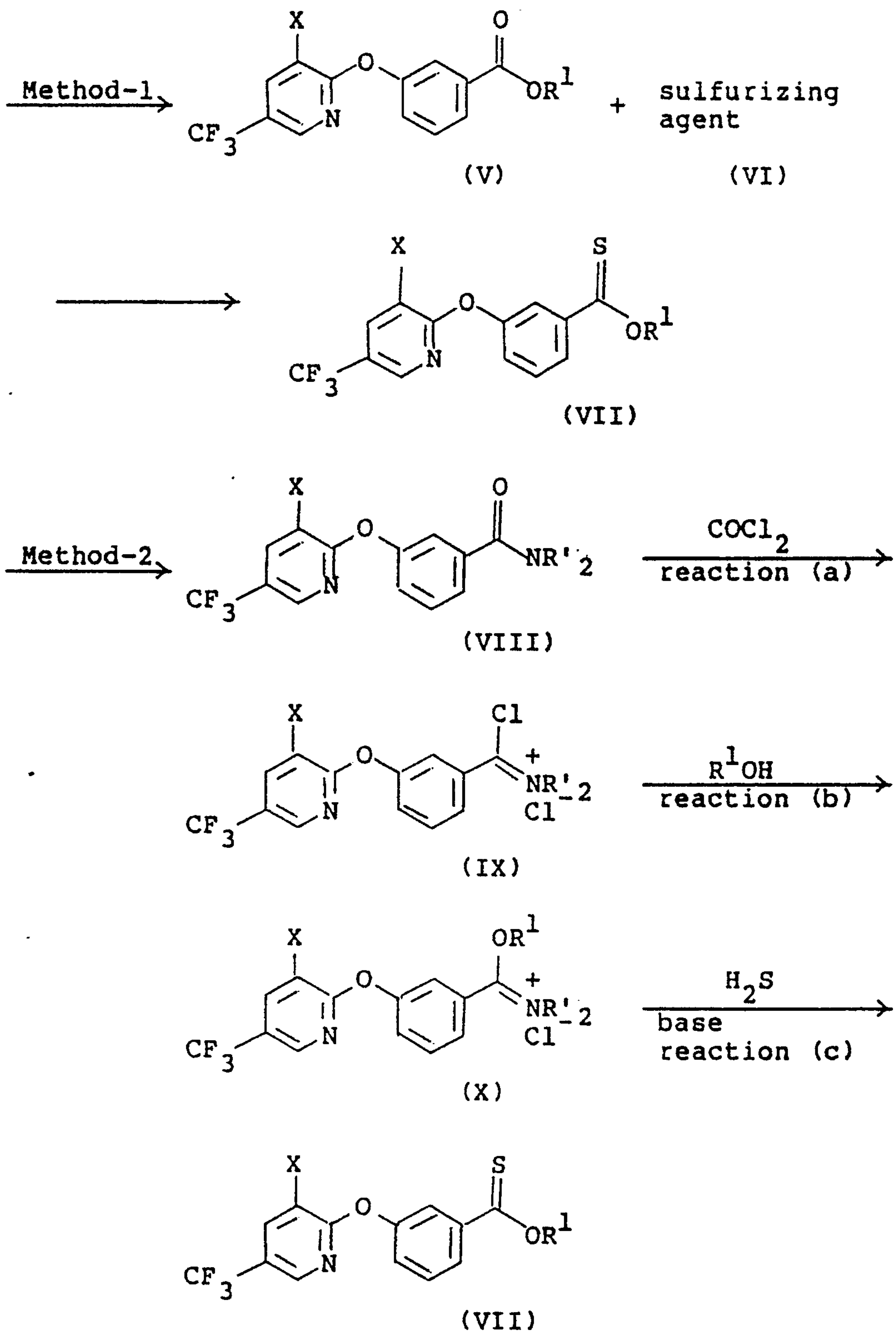


(V) (VI) (VII) (VIII) (IX) (X) (VII)

[2nd step]

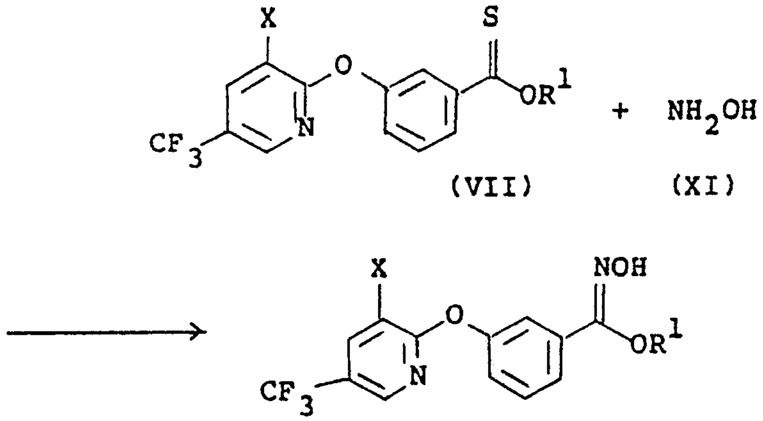

(VII) (XI)

(XII)

[3rd step]

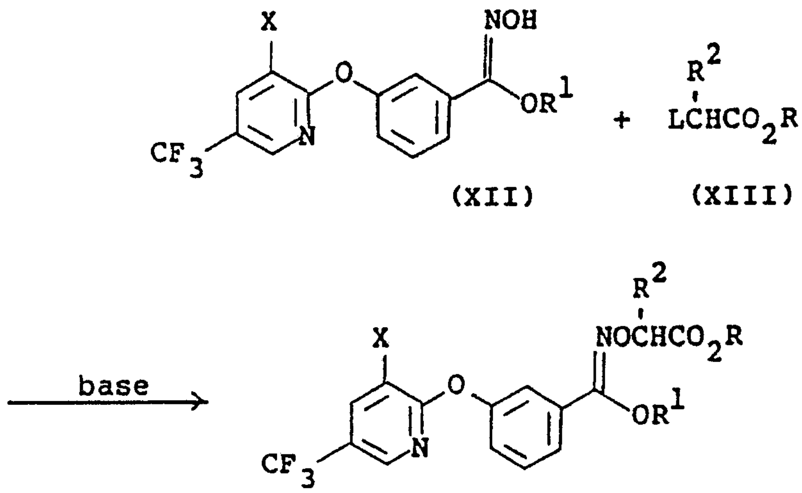


(XII) (XIII)

(XIV)

[4th step]

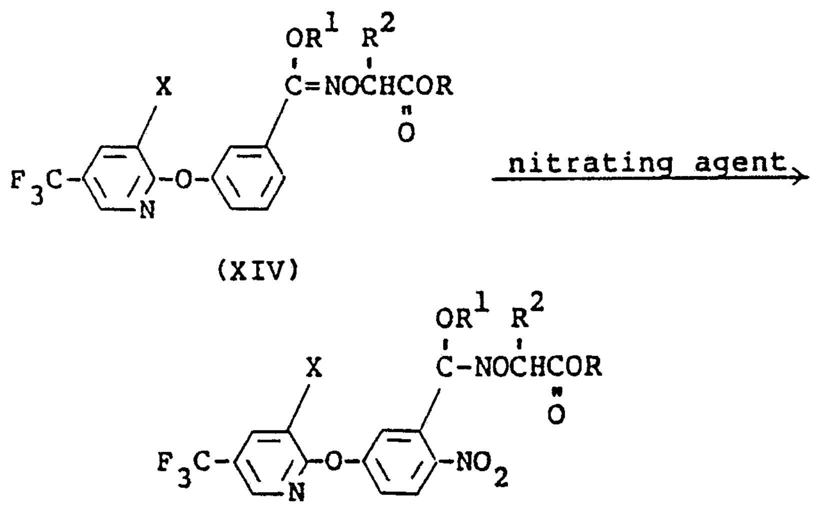


(XIV)

(I)

In the above formulae, R, $R^1$ and $R^2$ are as defined and L represents a leaving group.

The above steps 1 to 4 will be described below in detail.

First step (method-1)

In this step, the 3-(substituted pyridyloxy)benzoate derivative (V) is reacted with the sulfurizing agent (VI) in an inert solvent to give the compound (VII).

The sulfurizing agent (VI) used may be, for example, 2,4-bis(4-methoxyphenyl)-1,3-dithia-2,4-diphosphethane-2,4-disulfide, diethyl dithiophosphate and phosphorus pentasulfide.

The reaction is carried out usually at room temperature to the refluxing temperature of the solvent, preferably room temperature to about 150 °C, for a period of usually 1 hour to 10 days, preferably 3 hours to 7 days, using 1 to 50 moles, preferably 1 to 20 moles, of the sulfurizing agent (VI) per mole of the compound (V).

Examples of the inert solvent that can be used in this invention include aromatic hydrocarbons such as benzene, toluene and xylene, ethers such as tetrahydrofuran and dioxane, chlorobenzene, and acetonitrile.

After the reaction, the compound of formula (VII) can be isolated in a sufficient purity by, for example, evaporating the solvent and then treating the residue by column chromatography, or by adding a non-polar solvent such as pentane, hexane, or petroleum ether to the reaction mixture to form a precipitate, filtering the mixture, and concentrating the filtrate.

First step (method-2)

This step consists of reaction (a), reaction (b) and reaction (c).

The reaction (a) is carried out usually at room temperature to the refluxing temperature of the solvent, preferably room temperature to about 100 °C, for a period of usually 1 hour to 10 days, preferably 3 hours to 7 days, using 1 to 30 moles, preferably 2 to 20 moles, of phosgene per mole of the compound (VIII).

Examples of inert solvents that can be useed in this invention are halogenated hydrocarbons such as dichloromethane, dichloroethane and chloroform, aromatic hydrocarbons such as benzene, toluene and xylene, and ethers such as tetrahydrofuran and dioxane.

After the reaction, the intermediate (IX) may be isolated. Usually, the solvent and the excess of phosgene are evaporated from the reaction mixture, and the residue is submitted to the subsequent reaction (b).

The reactions (b) and (c) may be usually carried out continuously in the same solvent and the same reactor.

The reaction (b) is carried out at a temperature of usually about -50 °C to the refluxing temperature of the solvent, preferably about -20 °C to room temperature, usually 1 minute to 1 day, preferably 5 minutes to 1 hour.

In the reaction (c), triethylamine, pyridine, 2,6-lutidine and diazabicycloundecene, for example, may be used as bases. The base is used in an amount of 1 to 20 moles, preferably 2 to 10 moles, per mole of the compound (IX), and hydrogen sulfide is used in an amount of 1 to 50 moles, preferably 1 to 20 moles, per mole of the compound (IX). The reaction (c) is usually carried out at a temperature of about -50 °C to the refluxing temperature of the solvent, preferably about -20 °C to room temperature, for a period of 1 minute to 1 day, preferably 5 minutes to 1 hour.

Examples of inert solvents that can be used in this reaction include halogenated hydrocarbons such as dichloromethane, dichloroethane and chloroform, aromatic hydrocarbons such as benzene, toluene and xylene, ethers such as diethyl ether, tetrahydrofuran and dioxane, and mixtures of these.

After the reaction, the reaction mixture is subjected to a usual after-treatment, and the excess of the base is evaporated to isolate the compound (VII) in a sufficient purity.

Second step

In this step, the O-alkyl 3-(substituted pyridyloxy)thiobenzoate derivative (VII) is reacted with the hydroxylamine (XI) in an organic solvent to produce the compound (XII).

The reaction is carried out at a temperature of 0 (under cooling with ice) to 200 °C, preferably room

temperature to 120 °C, for a period of usually 5 minutes to 10 days, preferably 15 minutes to 3 days using generally 1 to 5 moles, preferably 1 to 2 moles, of the hydroxylamine (XI) per mole of the compound (VII).

Examples of the solvent that can be used in this invention include alcohols such as methanol and ethanol, aromatic hydrocarbons such as benzene, toluene and xylene, ethers such as ether, tetrahydrofuran and dioxane, halogenated hydrocarbons such as dichloromethane, chloroform and carbon tetrachloride, acetonitrile, dimethylformamide and dimethyl sulfoxide. A mixture of such an organic solvent with water may also be used.

When a salt of the hydroxylamine (XI) such as its hydrochloride, sulfate or oxalate is used, it is convenient to treat the hydroxylamine salt with a base such as sodium hydroxide, potassium hydroxide, sodium carbonate, potassium carbonate, sodium acetate, potassium acetate, sodium methoxide or sodium ethoxide prior to preparation of a solution of the hydroxylamine (XI).

After the reaction, the compound (XII) may be isolated from the reaction mixture by, for example, evaporating the solvent from it, and subjecting the residue to a treatment in a customary manner such as extraction with water and an organic solvent, re-crystallization or column chromatography.

Third step

In this step, the alkyl 3-(substituted pyridyloxy)benzohydroximate obtained in the second step is reacted with the compound (XIII) in the presence of a base in an organic solvent to produce the compound (XIV).

The action is carried out at a temperature of usually -50 °C to the refluxing temperature of the solvent, preferably -10 to about 80 °C, for a period of usually 0.5 to 20 hours using generally 1 to 5 moles, preferably 1 to 3 moles, of the compound (XIII) per mole of the compound (XII).

Examples of the leaving group L in the compound (XIII) may be, for example, chlorine, bromine, iodine, a benzenesulfonyloxy group, a p-toluenesulfonyloxy group, a methanesulfonyloxy group or a trifluoromethanesulfonyloxy group.

Examples of the solvent used in this reaction include alcohols such as methanol and ethanol, aromatic hydrocarbons such as benzene, toluene and xylene, ethers such at tetrahydrofuran and dioxane, halogenated hydrocarbons such as chloroform and methylene chloride, acetone, acetonitrile, dimethylformamide, dimethylsulfoxide and N-methylpyrrolidone. A mixture of such an organic solvent with water may also be used.

Examples of the base that can be used include sodium methoxide, sodium ethoxide, sodium hydride, potassium t-butoxide, sodium hydroxide, potassium hydroxide, sodium carbonate and potassium carbonate.

The reaction may also be carried out in a two-phase system. It is convenient in this case to use a phase transfer catalyst, for example a quaternary ammonium salt such as tetramethyl ammonium bromide, tetrabutyl ammonium bromide or benzyl tributyl ammonium bromide and a quaternary phosphonium salt such as tetraphenyl phosphonium bromide in an amount of, for example, 1 to 60 % by weight, preferably 5 to 40 % by weight.

After the reaction, the compound (XIV) may be isolated from the reaction mixture by, for example, pouring it into water and treating it in a customary manner such as extraction with an organic solvent, re-crystallization or column chromatography.

Fourth step

The nitration agent used for nitration of the compound (XIV) in this step may be an ordinary nitro compound such as nitric acid/sulfuric acid, sodium nitrate/sulfuric acid or potassium nitrate/sulfuric acid.

The amount of the nitrating agent used is not strictly limited. Generally, its suitable amount is 1 to 10 moles, preferaly 1 to 3 mloes, per mole of the compound of formula (XIV). The reaction temperature may be varied depending upon the type of the nitrating agent, and may gnerally be -20 to 100 °C, preferably -10 to 80 °C. The reaction can be terminated usually in about 0.5 to 5 hours at these temperatures.

The nitration reaction may be carried out in the absence of a solvent or in a solvent such as dichloromethane or dichloroethane.

After the reaction, the desired compound of formula (I) may be isolated in a good yield by, for example, pouring the reaction mixture into water, extracting it with an organic solvent and crystallizing the extract.

The following Examples illustrate the synthesis of the compound of this invention.

## EXAMPLE 1

Production of methyl O-methoxycarbonylmethyl-5-(3-chloro-5-trifluoromethyl-2-pyridyloxy)-2-nitrobenzohydroximate:-

36.2 g (0.10 mole) of 3-(3-chloro-5-trifluoromethyl-2-pyridyloxy)-2-nitrobenzoic acid was dissolved in 36.2 ml of thionyl chloride, and the solution was heated under reflux for 1.5 hours. The excess of thionyl chloride was evaporated to give an acid chloride.

A solution of the acid chloride in 150 ml of ether was added dropwise to a solution consisting of 10.5 g (0.1 mole) of methyl aminoxyacetate, 10.1 g (0.1 mole) of triethylamine and 300 ml of dry ether with stirring under ice cooling over the course of about 20 minutes. Thereafter, the mixture was stirred under ice cooling for 30 minutes and then at room temperature for 1.5 hours. The reaction solution was poured into 300 ml of ice water, and extracted with 200 ml of ethyl acetate three times. The organic layers were washed with a saturated aqueous solution of sodium chloride and dried over magnesium sulfate. The desiccant was separated by filtration and the solvent was evaporated. The resulting solid was recrysrtallized from toluene, to give 37.2 g (yield 82.7 %) of methoxycarbonylmethyl 5-(3-chloro-5-trifluoromethyl-2-pyridyloxy)-2-nitrobenzohydroxamate.

The resulting hydroxamate (37.2 g) was dissolved in a mixed solvent composed of 70 ml of ether and 70 ml of tetrahydrofuran, and with stirring under ice cooling, a solution of diazomethane prepared from 24.8 g (0.241 mole) of N-nitrosomethylurea in 300 ml of ether was added dropwise over about 40 minutes. The mixture was then stirred under ice cooling for about 30 minutes. The excess of diazomethane was decomposed with acetic acid, and the solvent was evaporated. The resulting solid was recrystallized from methanol to give 27.0 g (yield 70.4 %) of the desired compound No. 32 (syn-type). mp. 71.0 to 73.0 °C.

5.0 g of the above compound No. 32 was dissolved in 200 ml of toluene. The solution was bubbled with $N_2$, deaerated, and irradiated with a high-pressure mercury lamp (UVL-1009, made by Rikokagaku Sangyo) for 3 hours. The solvent was evaporated, and the residue was purified by column chromatography (silica gel; n-hexane/ethyl acetate (2:1) to give 3.5 g (yield 70.0 %) of an isomer (anti-type; mp. 84.5-85.5 °C) of compound No. 32.

## EXAMPLE 2

Production of ethyl O-methoxycarbonylmethyl-5-(3-chloro-5-trifluoromethyl-2-pyridyloxy)-2-nitrobenzohydroximate (compound No. 53):-

A suspension composed of 2.25 g (5 millimoles) of the hydroxamate obtained in the same way as in Example 1, 0.83 g (6 millimoles) of anhydrous potassium carbonate and 10 ml of DMF was heated to 65 to 67 °C, and 1.2 g (about 6 millimoles) of ethyl p-toluenesulfonate was added dropwise over the course of about 15 minutes. Then, the mixture was stirred at the same temperature as above for 1 hour. The reaction mixture was poured into about 150 ml of ice water, and extracted with 30 ml of ethyl acetate twice. The organic layers were washed with a saturated aqueous solution of sodium chloride, and then dried over magnesium sulfate. The desiccant was separated by filtration, and the solvent was evaporated. The resulting oily substance was purified by column chromatography (silica gel; n-hexane/ethyl acetate 4:1) to give 1.4 g (yield 58.5 %) of the desired compound No. 53. $n_D^{21.2}$ = 1.5359.

Table 1 shows pyridyl phenyl ether derivatives of the invention synthesized by techniques similar to those shown in the above Examples.

Table 1

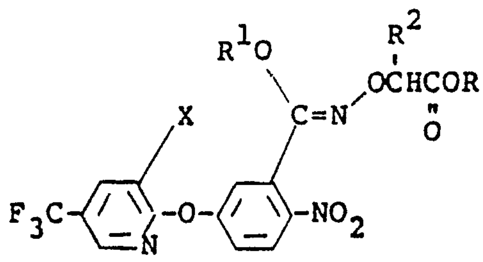

(syn-step)

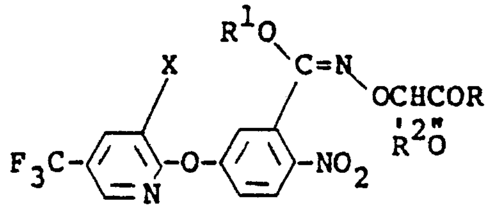

(anti-type)

| Compound No. | X | $R^1$ | $R^2$ | R | Properties |
|---|---|---|---|---|---|
| 1. | H | $CH_3$ | H | $CH_3$ | mp. 81.0-84.0 °C (syn-type) |
| 2. | H | $CH_3$ | H | $C_2H_5$ | |
| 3. | H | $CH_3$ | H | $n-C_3H_7$ | |
| 4. | H | $CH_3$ | H | $i-C_3H_7$ | |
| 5. | H | $CH_3$ | H | $n-C_4H_9$ | mp. 55.0-56.0 °C (anti-type) |
| 6. | H | $CH_3$ | H | $i-C_4H_9$ | |
| 7. | H | $CH_3$ | H | $s-C_4H_9$ | |
| 8. | H | $CH_3$ | H | $t-C_4H_9$ | |
| 9. | H | $CH_3$ | H | $n-C_5H_{11}$ | |
| 10. | H | $CH_3$ | H | $t-C_5H_{11}$ | |
| 11. | H | $CH_3$ | $CH_3$ | $CH_3$ | |
| 12. | H | $CH_3$ | $CH_3$ | $C_2H_5$ | |
| 13. | H | $CH_3$ | $CH_3$ | $n-C_3H_7$ | |
| 14. | H | $CH_3$ | $CH_3$ | $i-C_3H_7$ | |
| 15. | H | $CH_3$ | $CH_3$ | $n-C_4H_9$ | |
| 16. | H | $CH_3$ | CH3 | $t-C_4H_9$ | |
| 17. | H | $CH_3$ | $CH_3$ | $n-C_5H_{11}$ | |
| 18. | H | $CH_3$ | $C_2H_5$ | $CH_3$ | |

- to be continued -

Table 1 (continued)

| Compound No. | X | $R^1$ | $R^2$ | R | Properties |
|---|---|---|---|---|---|
| 19. | H | $CH_3$ | $C_2H_5$ | $C_2H_5$ | |
| 20. | H | $CH_3$ | $C_2H_5$ | $n-C_3H_7$ | |
| 21. | H | $CH_3$ | $C_2H_5$ | $n-C_4H_9$ | |
| 22. | H | $C_2H_5$ | H | $CH_3$ | |
| 23. | H | $C_2H_5$ | H | $C_2H_5$ | |
| 24. | H | $C_2H_5$ | H | $n-C_4H_9$ | |
| 25. | H | $i-C_3H_7$ | H | $CH_3$ | |
| 26. | H | $i-C_3H_7$ | H | $C_2H_5$ | |
| 27. | H | $i-C_3H_7$ | H | $n-C_4H_9$ | |
| 28. | H | $C_2H_5$ | $CH_3$ | $CH_3$ | |
| 29. | H | $C_2H_5$ | $CH_3$ | $n-C_4H_9$ | |
| 30. | H | $i-C_3H_7$ | $CH_3$ | $CH_3$ | |
| 31. | H | $i-C_3H_7$ | $CH_3$ | $n-C_4H_9$ | |
| 32. | Cl | $CH_3$ | H | $CH_3$ | mp. 71.0-73.0 °C (syn-type)<br>mp. 84.5-85.5 °C (anti-type) |
| 33. | Cl | $CH_3$ | H | $C_2H_5$ | mp. 101.5-103.0 °C (syn-type)<br>mp. 58.0-59.5 °C (anti-type) |
| 34. | Cl | $CH_3$ | H | $n-C_3H_7$ | mp. 71.5-73.0 °C (anti-type) |
| 35. | Cl | $CH_3$ | H | $i-C_3H_7$ | mp. 97.5-99.0 °C (anti-type) |
| 36. | Cl | $CH_3$ | H | $n-C_4H_9$ | mp. 84.0-85.0 °C (anti-type) |
| 37. | Cl | $CH_3$ | H | $i-C_4H_9$ | mp. 77.5-79.0 °C (anti-type) |

- to be continued -

Table 1 (continued)

| Compound No. | X | $R^1$ | $R^2$ | R | Properties |
|---|---|---|---|---|---|
| 38. | Cl | $CH_3$ | H | $s-C_4H_9$ | |
| 39. | Cl | $CH_3$ | H | $t-C_4H_9$ | |
| 40. | Cl | $CH_3$ | H | $n-C_5H_{11}$ | mp. 71.5-72.5 °C (anti-type) |
| 41. | Cl | $CH_3$ | H | $t-C_5H_{11}$ | |
| 42. | Cl | $CH_3$ | $CH_3$ | $CH_3$ | $n_D^{20.4}$ 1.5328 (Syn-type) |
| 43. | Cl | $CH_3$ | $CH_3$ | $C_2H_5$ | |
| 44. | Cl | $CH_3$ | $CH_3$ | $n-C_3H_7$ | |
| 45. | Cl | $CH_3$ | $CH_3$ | $i-C_3H_7$ | |
| 46. | Cl | $CH_3$ | $CH_3$ | $n-C_4H_9$ | |
| 47. | Cl | $CH_3$ | $CH_3$ | $t-C_4H_9$ | |
| 48. | Cl | $CH_3$ | $CH_3$ | $n-C_5H_{11}$ | |
| 49. | Cl | $CH_3$ | $C_2H_5$ | $CH_3$ | |
| 50. | Cl | $CH_3$ | $C_2H_5$ | $C_2H_5$ | |
| 51. | Cl | $CH_3$ | $C_2H_5$ | $n-C_3H_7$ | |
| 52. | Cl | $CH_3$ | $C_2H_5$ | $n-C_4H_9$ | |
| 53. | Cl | $C_2H_5$ | H | $CH_3$ | $n_D^{21.1}$ 1.5359 (Syn-type) |
| 54. | Cl | $C_2H_5$ | H | $C_2H_5$ | |
| 55. | Cl | $C_2H_5$ | H | $n-C_4H_9$ | |
| 56. | Cl | $i-C_3H_7$ | H | $CH_3$ | $n_D^{21.1}$ 1.5336 (Syn-type) |
| 57. | Cl | $i-C_3H_7$ | H | $C_2H_5$ | |
| 58. | Cl | $i-C_3H_7$ | H | $n-C_4H_9$ | |

- to be continued -

Table 1 (continued)

| Compound No. | X | $R^1$ | $R^2$ | R | Properties |
|---|---|---|---|---|---|
| 59. | Cl | $C_2H_5$ | $CH_3$ | $CH_3$ | |
| 60. | Cl | $C_2H_5$ | $CH_3$ | $n-C_4H_9$ | |
| 61. | Cl | $i-C_3H_7$ | $CH_3$ | $CH_3$ | |
| 62. | Cl | $i-C_3H_7$ | $CH_3$ | $n-C_4H_9$ | |
| 63. | F | $CH_3$ | H | $CH_3$ | |
| 64. | F | $CH_3$ | H | $C_2H_5$ | |
| 65. | F | $CH_3$ | H | $n-C_3H_7$ | |
| 66. | F | $CH_3$ | H | $i-C_3H_7$ | |
| 67. | F | $CH_3$ | H | $n-C_4H_9$ | |
| 68. | F | $CH_3$ | H | $t-C_4H_9$ | |
| 69. | F | $CH_3$ | H | $s-C_4H_9$ | |

## REFERENTIAL EXAMPLE 1

### Production of the compound (XIV):-

(A-1) Production of O-methyl 3-(3-chloro-5-trifluoromethyl-2-pyridyloxy)thiobenzoate (compound VIIa):-

24.5 g (0.074 mole) of methyl 3-(3-chloro-5-trifluoromethyl-2-pyridyloxy)benzoate was dissolved in 80 ml of xylene, and 40.0 g (0.099 mole) of 2,4-bis(4-methoxyphenyl)-1,3-dithia-2,4-diphosphethane-2,4-disulfide was added. The mixture was stirred at 120 °C for 20 hours, and xylene was evaporated. The residue was purified by column chromatography (silica gel; n-hexane/ether 98:2) to give 22.1 g (yield 86 %) of the desired compound VIIa.

$n_D^{22}$ = 1.5840

NMR [$CDCl_3$, δ (ppm)]: 4.29 (3H, s), 7.1-7.6 (2H, m), 7.9 -8.4 (4H, m).

(A-2) Production of O-methyl 3-(3-chloro-5-trifluoromethyl-2-pyridyloxy)thiobenzoate (compound VIIa):-

4.2 g (0.0122 mole) of N,N-dimethyl-3-(3-chloro-5-trifluoromethyl-2-pyridyloxy)benzamide was added to a methylene chloride solution (26 ml) of 8.2 g of phosgene at room temperature. The mixture was stirred at the same temperature for 18 hours. The excess of phosgene and methylene chloride were evaporated. The residue was dissolved in 26 ml of methylene chloride, and the solution was added dropwise to a THF (tetrahydrofuran) solution (20 ml) of 0.59 g (0.0128 mole) of ethanol. After the addition, the mixture was stirred at this temperature for 30 minutes, and while 4.3 ml (0.0532 mole) of pyridine was added dropwise at 5 to 10 °C, hydrogen sulfide was blown into the solution until it was saturated. The mixture was further stirred at this temperature for 15 minutes. The solvent was evaporated, and 30 ml of water was added. The mixture was extracted with 30 ml of ether three times. The organic layers were dried over magnesium sulfate. The desiccant was separated by filtration, and the solvent and pyridine were evaporated to give 4.0 g (yield 91 %) of the desired compound (VIIa).

(B) Production of methyl 3-(3-chloro-5-trifluoromethyl-2-pyridyloxy)benzohydroxamate (compound XIIa):-

1.53 g (0.022 mole) of hydroxylamine hydrochloride was dissolved in 21 ml of methanol, and 4.24 g (0.0922 mole) of a 28% methanol solution of sodium methoxide was added to the solution under ice cooling. The mixture was stirred at this temperature for 15 minutes, and suction-filtered to give a methanol solution of hydroxylamine.

The methanol solution (15 ml) of hydroxylamine obtained above was added to 6.61 g (0.019 mole) of O-methyl 3-(3-chloro-5-trifluoromethyl-2-pyridyloxy)thiobenzoate, and the mixture was stirred at the refluxing temperature for 1.5 hours. The solvent was evaporated, and the resulting oily mixture was purified by column chromatography (silica gel; n-hexane/ethyl acetate = 4:1) to give 5.73 g (yield 87 %) of the desired compound (XIIa) (syn:anti = about 4:6).
$n_D^{20}$ = 1.5545
NMR [$CDCl_3$, δ (ppm)]: 3.80, 4.01 (3H, s), 7.0-8.4 (6H, m).

(C-1) Production of methyl O-ethoxycarbonylmethyl-3-(3-chloro-5-trifluoromethyl-2-pyridyloxy)-benzohydoximate (compound XIVa):-

0.4 g (0.011 mole) of sodium hydride (60% in oil) was added little by little to a DMF (30 ml) solution of 3.47 g (0.01 mole) of methyl 3-(3-chloro-5-trifluoromethyl-2-pyridyloxy)benzohydroximate. The mixture was stirred at room temperature for 30 minutes, and 1.34 g (0.011 mole) of ethyl bromoacetate was added to the resulting solution at room temperature. The mixture was stirred at room temperature for 3 hours. The reaction mixture was poured into 60 ml of ice water, and extracted with 40 ml of ether twice. The organic layers were washed with a saturated aqueous solution of sodium chloride and dried over magnesium sulfate. The desiccant was separated by filtration, and the solvent was evaporated. The resulting oily substance was purified by column chromatography (silica gel; n-hexane/ethyl acetate = 5:1) to give 3.94 g (yield 91 %) of the desired compound (XIVa) (syn:anti = about 4:6).

This compound was carefully purified by column chromatography (silica gel; n-hexane/chloroform = 2:3) to give an anti-type compound (XIVc) and a syn-type compound (XIVb).

By the same method as in (C-1), compounds of formula (XIV) shown in Table 2 below were synthesized.

Table 2

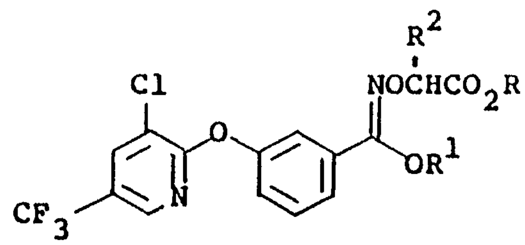

(XIV)

| Compound No. | $R^1$ | $R^2$ | R | Refractive index $[n_D^{20}]$ (melting point) | NMR δ (ppm) [in $CDCl_3$] | Syn, anti |
|---|---|---|---|---|---|---|
| XIVa | $CH_3$ | H | $CH_3$ | 1.5314 | 3.74, 3.77 (3H, s), 3.78, 4.10 (3H, s), 4.53, 4.61 (2H, s), 7.0-8.3 (6H, m) | mixture |
| XIVb | $CH_3$ | H | $CH_2CH_3$ | 1.5302 | 1.23 (3H, t, J=7Hz), 4.14 (3H, s), 4.21 (2H, q, J=7Hz), 4.60 (2H, s), 7.0-8.3 (6H, m) | syn |
| XIVc | $CH_3$ | H | $CH_2CH_3$ | 1.5210 | 1.25 (3H, t, J=7Hz), 3.78 (3H, s), 4.21 (2H, q, J=7Hz), 4.52 (2H, s), 7.0-8.3 (6H, m) | anti |
| XIVd | $CH_3$ | $CH_3$ | $CH_2CH_3$ | 1.5228 | 1.27 (3H, t, J=7Hz), 1.43, 1.52 (3H, q, J=7Hz), 3.77, 4.13 (3H, s), 4.20 (2H, q, J=7Hz), 4.59, 4.66 (1H, q, J=7Hz), 7.0-8.3 (6H, m) | mixture |
| XIVe | $CH_3$ | H | $CH_2CH_3$ | | 1.23, 1.25 (3H, t, J=7Hz), 3.78, 4.14 (3H, s), 4.21 (2H, q, J=7Hz), 4,52, 4.60 (2H, s), 7.0-8.3 (6H, m) | mixture |

(C-2) Production of methyl O-methoxycarbonylmethyl-3-(3-chloro-5-trifluoromethyl-2-pyridyloxy)-benzohydroximate (compound XIVa):-

Benzyl triethyl ammonium chloride (0.20 g; 0.88 millimole) was added to a solution of 1.04 g (3 millimoles) of methyl 3-(3-chloro-5-trifluoromethyl-2-pyridyloxy)benzohydroximate in 4.5 ml of methylene chloride, and the mixture was cooled. At -5 to 0 °C, 1.72 ml of a 50 % aqueous solution of sodium hydroxide was added dropwise. Then, at the same temperature, 0.92 g (6 millimoles) of methyl bromoacetate was added dropwise. The mixture was stirred at the same temperature for 20 minutes. The reaction mixture was poured into 30 ml of ice water, neutralized with dilute hydrochloric acid and extracted with 20 ml of ether three times. The organic layers were washed with a saturated aqueous solution of sodium chloride, and dried over magnesium sulfate. The desiccant was separated by filtration, and the solvent was evaporated. The resulting oily substance was purified by column chromatography (silica gel; n-hexane/ethyl acetate = 5:1) to give 1.17 g (yield 90 %) of the desired compound (XIVa) (syn:anti = about 4:6).

## EXAMPLE 3

Production of methyl O-methoxycarbonylmethyl 5-(3-chloro-5-trifluoromethyl-2-pyridyloxy)-2-nitrobenzohydroximate (compound No. 32):-

Sulfuric acid (1.31 g) was added at -10 °C to a solution of 0.47 g (1.12 millimoles) of methyl O-methoxycarbonylmethyl-3-(3-chloro-5-trifluoromethyl-2-pyridyloxy)benzohydroximate (syn:anti = about 4:6) in 0.71 ml of dicloroethane. Potassium nitrate was added little by little at -2 to 5 °C to the resulting solution. The mixture was stirred at the same temperature for 2 hours. The reaction mixture was poured into 10 ml of ice water, and extracted with 10 ml of dichloromethane twice. The organic layers were washed with water and a saturated aqueous solution of sodium chloride, and dried over magnesium sulfate. The desiccant was separated by filtration and the solvent was evaporated. The resulting oily substance (syn:anti = about 10:1) was crystallized with methanol to give 0.31 g (yield 60 %) of the desired compound No. 32 (syn-type).

Table 3 below shows compounds (I) prpeared by the same method as in Example 3.

Table 3

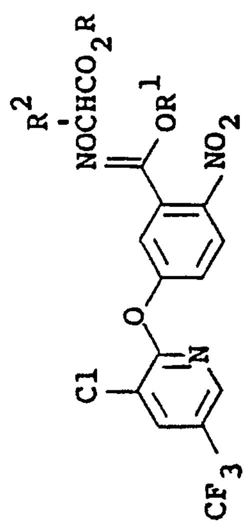

(I)

| Compound No. | $R^1$ | $R^2$ | R | Refractive index $[n_D^{20}]$ (melting point) | NMR δ (ppm) [in $CDCl_3$] |
|---|---|---|---|---|---|
| 32 | $CH_3$ | H | $CH_3$ | 71.0-73.0 °C | 3.75 (3H, s), 3.94 (3H, s), 4.55 (2H, s), 7.1-7.6 (2H, m), 7.9-8.4 (3H, m) |
| 33 | $CH_3$ | H | $CH_2CH_3$ | 101.5-103 °C | 1.28 (3H, t, J=7Hz), 3.95 (3H, s), 4.20 (2H, q, J=7Hz), 4.53 (2H, s), 7.1-7.5 (2H, m), 7.9-8.4 (3H, m) |

The compounds of general formula (I) provided by this invention have a broad herbicidal spectrum and very high herbicidal activity and show an excellent herbicidal efficacy in low dosages. They are useful as a foliar treating-type non-selective herbicide. When they are used in lower dosages than those which show non-selective activity, the compounds of this invention show good selectivity for cultivated crops in foliar treatment and can be an agriculturally useful selective herbicide.

The compounds of this invention can control various weeds growing in non-crop lands and croplands,

for example terrestrial narrow-leaved and broad-leaved weeds such as barnyardgrass (Echinochloa crus-galli), large crabgrass (Digitaria sanguinalis), green foxtail (Setaria viridis), goosegrass (Eleusine indica), (Paspalum thunbergii), annual bluegrass (Poa annua), amaranths, polygonums, goosefoot (Chenopodium album), lamb's-quarters (Chenopodium album), velvetleaf (Abutilon theophrasti), morning glories, cocklebur (Xanthnium strumarium), common ragweed (Ambrosia artemisiifolia), common purslane (Portulaca oleracea), devil's beggarticks (Bidens frondosa), sickledot (Cassia obtusifolia), black nightshade (Solanum nigrum), prickly sida (Sida spinosa), creeping woodsorrel (Oxalis corniculata), jimsonweed (Datura stramonium), red clover (Trifolium prastens), chickweed (Stellaria media), Japanese pealwort (Sagina japonica), shepherd's purse (Capsella bursa-pastoris), bittercress (Cardamine flexuosa), bedstraw (Galium spurium), poppy (Papaver spp.), scented mayweed (Matricaria chamommila), Canada thistle (Cirsium arvens), marigold, henbit (Lamium amplexicaule), (Veronica caninotesticulata), annual fleabane (Erigeron annus), Phyladelphia fleabane (Erigeron philadelphicus), horseweed (Erigeron canadensis), and hairy fleabane (Erigeron bonariensis); and aquatic narrowleaved and broadleaved plants such as barnyardgrass (Echinochloa crus-galli), umbrella plant (Cyperus difformis), monochoria (Monochoria vaginalis), false-pimpernel (Lindernia pyxidaria), American waterwort (Elatine triandra), Kikashigusa (Rotala indica), bulrush (Eleocharis juncoides), and spikerush (Eleocharis acicularis).

The compounds of this invention can be used by broadcast treatment before emergence of useful plants or by a direct spray method designed to avoid contact with leaves and stalks of useful plants in the growing stage after emergence not only in non-crop lands but also in croplands where the useful plants are cultivated. What is agriculturally useful is that when the compound of this invention is used in lower dosages than those which show non-selective activity, it shows good selectivity for cultivated crops even in a broadcast foliar treatment, and controls various weeds without any significant phytotoxicity to gramineous crops, particularly rice, corn, wheat, barley, sorghum and sugarcane, and broadleaved crops such as soybean and sunflower.

For actual use as a herbicide, the compound of this invention is mixed with agriculturally and horticulturally acceptable carriers or diluents, additives and auxiliary agents by a known method and used in usual formulations of agricultural chemicals such as a dust, granules, a wettable powder, an emulsifiable concentrate, a soluble powder or a sol. It may also be used as a mixture, or in combination, with other agricultural chemicals such as a fungicide, an insecticide, a miticide, another herbicide, a plant growth regulator, a fertilizer, and a soil conditioner.

By using it with another herbicide, the dosage can be reduced and the labor can be saved. In addition, by the synergistic action of the two types of herbicides, the herbicidal spectrum can be broadened, and a higher efficacy owing to the synergistic action can be expected

The carrier or diluent may be any solid or liquid carriers or diluents generally used in the field of agriculture. Solid carriers include talc or clays typified by kaolinite, montmorillonite and attapulgite; inorganic materials such as mica, pyrophyllite, pumice, vermicullite, gypsum, calcium carbonate, dolomite, diatomaceous earth, magnesium lime, apatite, zeolite, silicic anhydride and synthetic calcium silicate; organic substances of the plant origin such as soybean meal, tobacco powder, walnut powder, wheat flour, wood powder, starch and crystalline cellulose; natural or synthetic polymeric compounds such as coumarone resins, petroleum resins, alkyd resins, polyvinyl chloride, polyalkylene glycols, ketone resins, ester gum, copal gum and dammar gum; waxes such as carnauba wax and beeswax; and urea. Suitable liquid carriers or diluents include paraffinic or naphthenic hydrocarbons such as kerosene, mineral oils, spindile oil and white oil; aromatic hydrocarbons such as benzene, toluene, xylene, ethylbenzene, cumene and methylnaphthalene; chlorinated hydrocarbons such as carbon tetrachloride, chloroform, trichloroethylene, monochlorobenzene and o-chlorotoluene; ethers such as dioxane and tetrahydrofuran; ketones such as acetone, methyl ethyl ketone, diisobutyl ketone, cyclohexanone, acetophenone and isophorone; esters such as ethyl acetate, amyl acetate, ethylene glycol acetate, diethylene glycol acetate, dibutyl maleate and diethyl succinate; alcohols such as methanol, n-hexanol, ethylene glycol, diethylene glycol, cyclohexanol and benzyl alcohol; ether alcohols such as ethylene glycol ethyl ether, ethylene glycol phenyl ether, diethylene glycol ethyl ether and diethylene glycol butyl ether; polar solvents such as dimethyl formamide and dimethyl sulfoxide; and water.

Surface-active agents and other adjuvants may be used to emulsify, disperse, wet, spread or bind the compound of this invention, adjust its disintegrability, stabilize the active ingredients of the herbicide, improve the flowability of the herbicide, prevent corrosion, or otherwise. The surface-active agents may be nonionic, anionic, cationic and amphoteric. Usually, nonionic and anionic surface-active agents are suitable. Suitable nonionic surface-active agents include, for example, polyaddition products of ethylene oxide with higher alcohols such as lauryl alcohol, stearyl alcohol and oleyl alcohol; polyaddition products of ethylene oxide with alkylphenols such as isooctylphenol and nonylphenol; polyaddition products of ethylene oxides

with alkylnaphthols such as butylnaphthol and octylnaphthol; polyaddiion products of ethylene oxide with higher fatty acids such as palmitic acid, stearic acid and oleic acid; polyaddition products of ethylene oxide with mono- or di-alkylphosphoric acids such as stearylphosphoric acid and dilaurylphosphoric acid; polyaddition products of ethylene oxide with amine compounds such as dodecylamine and stearamide; higher fatty acid esters of polyhydric alcohols such as sorbitan, and polyaddition products of ethylene oxide with these esters; and a polyaddition product of ethylene oxide with propylene oxide. Suiable anionic surface-active agents include, for example, alkylsulfuric ester salts such as sodium laurylsulfate and oleyl sulfate amine salt; alkylsulfonic acid salts such as sodium 2-ethylhexenesulfonate; and arylsulfonic acid salts such as sodium isopropylnaphthalenesulfonate, sodium methylenebisnaphthalenesulfonate, sodium ligninsulfonate and sodium dodecylbenzenesulfonate.

To improve the properties of the formulated herbicide and increase its herbicidal effect, the compound of the invention may also be used in combination with other adjuvants including such polymeric compounds as casein, gelatin, albumin, glue, sodium alginate, carboxymethyl cellulose, methyl cellulose, hydroxyethyl cellulose and polyvinyl alcohol.

The above carriers or diluents and various adjuvants may be used singly in in suitable combinations depending upon the form of the formulated herbicide, the place and time of application, etc.

The proportion of the compound of the invention as an active ingredient in the formulated herbicide may be varied with the form of the herbicide. For example, it is usually from 0.1 to 99 % by weight, preferably from 1 to 80 % by weight.

The dust usually contains 1 to 25 % by weight of the active ingredient and the remainder being a solid carrier.

The wettable powder usually contains 25 to 90 % by weight of the active ingredient and the remainder being a solid carrier and a dispersing and wetting agent and optionally a protective colloid, a thixotropic agent, a defoamer, etc.

The granules usually contain 1 to 35 % by weight of the active ingredient and the remainder being mostly a solid carrier. The active ingredient is uniformly mixed with the solid carrier, or uniformly fixed to, or adsorbed on, the surface of the solid carrier. The granules have a particle diameter of about 0.2 to 1.5 mm.

The emulsifiable concentrate usually contains 1 to 30 % by weight of the active ingredient, about 5 to 20 % by weight of an emulsifier and the remainder being a liquid carrier and optionally a corrosion inhibitor.

The compound of formula (I) provided by this invention may be applied directly as a herbicide or in any desired form such as the formulations described above.

The herbicide of this invention can be applied to various weeds in the pre-emergence period to the growing period after emergence, preferably to those in the growing period, in non-crop lands and croplands. The weeds can be controlled by applying a herbicidally effective amount of the compound of the invention to the soil in a locus where pre-emergence weed control is desired, or by contacting it directly with weeds after emergence.

The rate of application may be small because the compound of the invention has very high herbicidal activity, and may be properly varied depending upon the types of weeds to be controlled, their growth stage, the place of application, the time of application, weather, etc. Generally, the rate of application is about 0.01 to 10,000 g per ha, preferably about 0.1 to 5,000 g per ha, as the amount of the compound of formula (I) (the active ingredient).

Preferably, it is about 50 to 2,000 g/ha for a non-selective herbicide, and about 1 to 50 g/ha for a selective herbicide.

The following Formulation Examples show some examples of formulating the compound of this invention. All parts herein are by weight.

FORMULATION EXAMPLE 1

Granules:-

| Ingredient | Parts |
|---|---|
| Compound No. 32 | 5 |
| Bentonite | 50 |
| Talc | 40 |
| Sodium dodecylbenzenesulfonate | 2 |
| Sodium ligninsulfonate | 2 |
| Polyoxyethylene alkyl aryl ether | 1 |

The above ingredients were thoroughly mixed, and a suitable amount of water was then added. The mixture was kneaded and granulated by a granulator to give 100 parts of granules.

| FORMULATION EXAMPLE 2 | |
|---|---|
| Wettable powder:- | |
| Ingredient | Parts |
| Compound No. 33 | 20 |
| Clay | 70 |
| White carbon | 5 |
| Sodium ligninsulfonate | 3 |
| Sodium dodecylbenzenesulfonate | 2 |

The above ingredients were mixed and kneaded and pulverized uniformly by a kneader to give 100 parts of a wettable powder.

| FORMULATION EXAMPLE 3 | |
|---|---|
| Emulsifiable concentrate:- | |
| Ingredient | Parts |
| Compound No. 33 | 30 |
| Cyclohexanone | 70 |
| Calcium dodecylbenzenesulfonate | 5 |
| Polyoxyethylene aklkyl aryl ether | 5 |

The above ingredients were uniformly mixed and dissolved to form 100 parts of an emulsifiable concentrate.

| FORMULATION EXAMPLE 4 | |
|---|---|
| Flowable agent:- | |
| Ingredient | Parts |
| Compound No. 1 | 20 |
| Polyoxyethylene alkyl aryl ether | 2 |
| Calcium dodecylbenzenesulfonate | 3 |
| Silicone-type defoamer | 0.2 |
| Propylene glycol | 8 |
| 2% Aqueous solution of guar gum | 12 |
| Water (remainder) | 54.8 |

The above ingredients were mixed and pulverized uniformly by a wet pulverizer to give 100 parts of a flowable agent.

Other forms of the formulation of the compound of this invention could be prepared substantially in accordance with the above Formulation Examples.

The following Test Examples demonstrate the excellent herbicidal activity of the compounds of formula (I) provided by this invention.

TEST EXAMPLE 1

Foliar treatment:-

Upland farm soil was filled in square pots (30 x 30 x 8 cm). Seeds of various weeds indicated in Table 4 were sown in fixed amounts, and grown in a greenhouse until the plants grew to the 3- to 6-leaf stage (27th day after sowing).

Each of the test compounds prepared as in Formulation Example 3 was diluted in water containing 0.25 % of "Surfactant WK" (a product of Kao Co., Ltd.) as a spreader so that the amount of the active ingredient was as shown in Table 4. The diluted chemical was applied uniformly to the foliage of the plants in an amount corresponding to 500 liters/ha.

Twenty-one days after the application, the herbicidal effect on the weeds was rated on the following standards, and the results are shown in Table 4.

| Rating Standards (11 rates) | | |
|---|---|---|
| Rating | Herbicidal effect (herbicidal rate, %, based on a non-treated area) | Phytotoxicity to crops (phytotoxicity rate, %, based on a non-treated area) |
| 0 | 0 | Same as left |
| 1 | over 0 to 10 | |
| 2 | over 10 to 20 | |
| 3 | over 20 to 30 | |
| 4 | over 30 to 40 | |
| 5 | over 40 to 50 | |
| 6 | over 50 to 60 | |
| 7 | over 60 to 70 | |
| 8 | over 70 to 80 | |
| 9 | over 80 to 90 | |
| 10 | over 90 to 100 (withered) | |

Table 4

| Test compound | Amount of the active ingredient (g/ha) | Herbicidal effect | | | | | | | | | | | |
|---|---|---|---|---|---|---|---|---|---|---|---|---|---|
| | | a | b | c | d | e | f | g | h | i | j | k | ℓ |
| No. 32 (mp. 71.0--73.0 °C) | 31 | 3 | 6 | 9 | 8 | 5 | 10 | 8 | 10 | 10 | 10 | 8 | 4 |
| | 63 | 9 | 10 | 10 | 9 | 8 | 10 | 10 | 10 | 10 | 10 | 9 | 5 |
| | 125 | 9 | 10 | 10 | 10 | 9 | 10 | 10 | 10 | 10 | 10 | 10 | 9 |
| | 250 | 10 | 10 | 10 | 10 | 10 | 10 | 10 | 10 | 10 | 10 | 10 | 10 |
| No. 32 (mp. 84.5--85.5 °C) | 31 | 4 | 6 | 9 | 8 | 5 | 10 | 8 | 10 | 10 | 10 | 7 | 5 |
| | 63 | 9 | 10 | 10 | 9 | 8 | 10 | 10 | 10 | 10 | 10 | 9 | 5 |
| | 125 | 10 | 10 | 10 | 10 | 9 | 10 | 10 | 10 | 10 | 10 | 10 | 9 |
| | 250 | 10 | 10 | 10 | 10 | 10 | 10 | 10 | 10 | 10 | 10 | 10 | 10 |
| No. 33 (mp. 58.0--59.5 °C) | 31 | 5 | 7 | 9 | 9 | 6 | 10 | 8 | 10 | 10 | 9 | 9 | 5 |
| | 63 | 9 | 10 | 10 | 9 | 9 | 10 | 10 | 10 | 10 | 10 | 10 | 7 |
| | 125 | 10 | 10 | 10 | 10 | 10 | 10 | 10 | 10 | 10 | 10 | 10 | 9 |
| | 250 | 10 | 10 | 10 | 10 | 10 | 10 | 10 | 10 | 10 | 10 | 10 | 10 |

Table 4 (continued)

| Test compound | Amount of the active ingredient (g/ha) | Herbicidal effect | | | | | | | | | | | |
|---|---|---|---|---|---|---|---|---|---|---|---|---|---|
| | | a | b | c | d | e | f | g | h | i | j | k | ℓ |
| No. 33 (mp. 101.5-103.0 °C) | 31 | 4 | 8 | 9 | 9 | 6 | 10 | 7 | 10 | 10 | 8 | 9 | 5 |
| | 63 | 9 | 10 | 10 | 10 | 8 | 10 | 10 | 10 | 10 | 9 | 10 | 7 |
| | 125 | 10 | 10 | 10 | 10 | 10 | 10 | 10 | 10 | 10 | 10 | 10 | 10 |
| | 250 | 10 | 10 | 10 | 10 | 10 | 10 | 10 | 10 | 10 | 10 | 10 | 10 |
| Comparison (a*) "paraquat" | 125 | 2 | 5 | 10 | 10 | 10 | 10 | 1 | 10 | 1 | 0 | 8 | 9 |
| | 250 | 8 | 10 | 10 | 10 | 10 | 10 | 3 | 10 | 2 | 1 | 10 | 10 |
| | 500 | 9 | 10 | 10 | 10 | 10 | 10 | 10 | 10 | 8 | 10 | 10 | 10 |
| | 750 | 10 | 10 | 10 | 10 | 10 | 10 | 10 | 10 | 10 | 10 | 10 | 10 |
| Non-treated | | 0 | 0 | 0 | 0 | 0 | 0 | 0 | 0 | 0 | 0 | 0 | 0 |

* a: 1,1′-dimethyl-4,4′-bipyridium dichloride

In Table 4, a, b, c, d, e, f, g, h, i, j, k and ℓ represent the following weeds.

a: barnyard grass
b: fingergrass
c: giant foxtail
d: johnsongrass
e: annual bluegrass
f: green amaranth
g: ivyleaf morningglory (Ipomoea hederacea)

h: jimsonweed
i: pale smartweed
j: lamb's-quaters
k: cocklebur
ℓ: common ragweed

TEST EXAMPLE 2

Safety to useful plants cropped after chemical treatment:-

An upland farm in which naturally occurrng weeds such as green amaranth (Amaranthus reroflexus), pale smartweed (Polygonum lapathifolium), velvetleaf, barnyardgrass, fingergrass and giant foxtail (Setaria faberi) was divided into areas each having an area of 10 $m^2$ (2 x 5 m). A fixed rate of the compound of this invention formulated and then diluted as in Test Example 1 was uniformly sprayed onto each of the test areas in an amount corresponding to 500 liters/ha of water.

After the lapse of 1 week from the chemical treatment, the surface layer, 10 cm deep, of the soil in each of the areas which were almost a bare land as a result of killing the naturally occurring weeds was fully mixed up, and immediately then, seeds of each of the crops shown in Table 5 were sown. Their growth was examined.

On the 30th day after sowing, the degree of phytotoxicity was rated in accordance with the standards given in Test Example 1. The results are shown in Table 5.

In Table 5, A, B, C, D, E, and F represent the following crops.

A: corn
B: wheat
C: rice
D: soybean
E: cotton
F: sunflower

Table 5

| Test compound | Amount of the active ingredient (g/ha) | Phytotoxicity to crops | | | | | |
|---|---|---|---|---|---|---|---|
| | | A | B | C | D | E | F |
| No. 32 (mp. 71.0-73.0 °C) | 125<br>250<br>500 | 0<br>0<br>0 | 0<br>0<br>0 | 0<br>0<br>0 | 0<br>0<br>0 | 0<br>0<br>0 | 0<br>0<br>0 |
| No. 33 (mp. 58.0-59.5 °C) | 125<br>250<br>500 | 0<br>0<br>0 | 0<br>0<br>0 | 0<br>0<br>0 | 0<br>0<br>0 | 0<br>0<br>0 | 0<br>0<br>0 |
| No. 33 (mp. 101.5-103.0 °C) | 125<br>250<br>500 | 0<br>0<br>0 | 0<br>0<br>0 | 0<br>0<br>0 | 0<br>0<br>0 | 0<br>0<br>0 | 0<br>0<br>0 |
| a* | 500 | 0 | 0 | 0 | 0 | 0 | 0 |
| Non-treated | | 0 | 0 | 0 | 0 | 0 | 0 |

* Same as the footnote to Table 4 (paraquat).

## Claims

1. A compound of the formula

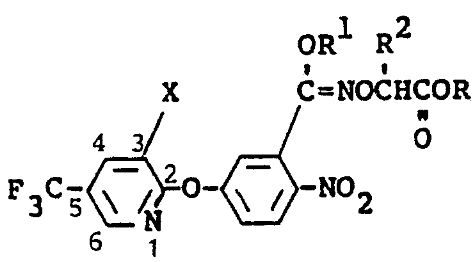

.... (I)

in which R and $R^1$ each represent a lowre alkyl group, $R^2$ represents a hydrogen atom or a lower alkyl group, and X represents a hydrogen or a halogen atom.

2. The compound of claim 1 in which $R^1$ represents a methyl group and $R^2$ represents a hydrogen atom.

3. The compound of claim 2 in which R represents a methyl or ethyl group.

4. The compound of claim 1 in which X represents a chlorine atom.

5. The compound of claim 1 which is methyl O-methoxycarbonylmethyl-5-(3-chloro-5-trifluoromethyl-2-pyridyloxy)-2-nitrobenzohydroximate or methyl O-ethoxycarbonylmethyl-5-(3-chloro-5-trifluoromethyl-2-pyridyloxy)-2-nitrobenzohydroximate.

6. A process for producing the compound of formula (I) claimed in claim 1, which comprises nitrating a compound of the formula

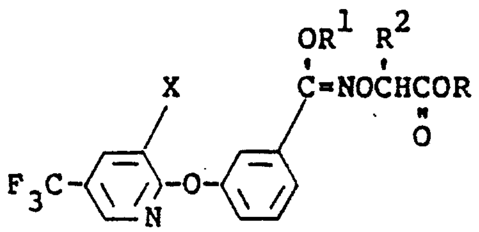

in which R, $R^1$, $R^2$ and X are as defined in claim 1.

7. A herbicidal composition comprising a compound of formula (I) set forth in claim 1 and an agriculturally and horticulturally acceptable carrier or diluent.

8. A herbicide comprising a compound of formula (I) set forth in claim 1.

9. A method of controlling weeds which comprises applying a compound of formula (I) set forth in claim 1 to the locus where the weeds are to be controlled, or to the weeds to be controlled.

10. Use of a compound of formula (I) set forth in claim 1 as a herbicide.

Claims for the following contracting State: ES

1. A process for producing a compound of the formula

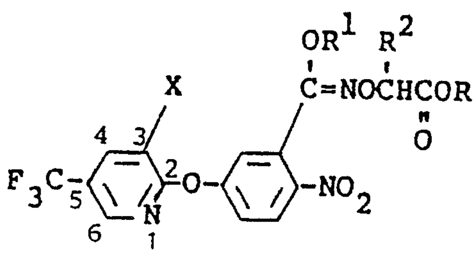

... (I)

in which R and $R^1$ each represent a lower alkyl group, $R^2$ represents a hydrogen atom or a lower alkyl group, and X represents a hydrogen or a halogen atom, which comprises nitrating a compound of the formula

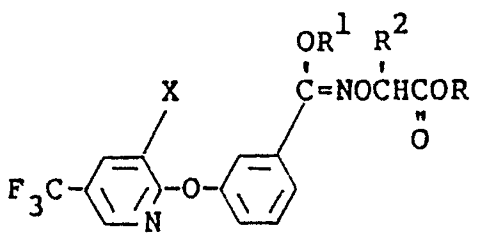

in which R, $R^1$, $R^2$ and X are as defined above.

2. A herbicidal composition comprising a compound of formula (I) set forth in claim 1 and an agriculturally and horticulturally acceptable carrier or diluent.

3. A herbicide comprising a compound of formula (I) set forth in claim 1.

4. A method of controlling weeds which comprises applying a compound of formula (I) set forth in claim 1 to the lo cus where the weeds are to be controlled, or to the weeds to be controlled.

5. Use of a compound of formula (I) set forth in claim 1 as a herbicide.

European Patent Office

# EUROPEAN SEARCH REPORT

Application number

EP 88110942.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | EP - A2/A3 - 0 155 613 (PPG IND.) * Claims 1,4,5 * | 1,7-9 | C 07 D 213/64<br>A 01 N 43/40 |
| A | US - A - 4 539 039 (SUDARSHAN K. MALHOTRA) * Claims 1,3,5 * | 1,7-9 | |
| A | EP - A1 - 0 023 891 (CIBA-GEIGY) * Claims 1,3,6,8,9 * | 1,4,6,7,10 | |

TECHNICAL FIELDS SEARCHED (Int. Cl.4)

C 07 D 213/00

The present search report has been drawn up for all claims

| Place of search | Date of completion of the search | Examiner |
|---|---|---|
| VIENNA | 21-10-1988 | HOCHHAUSER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82